# EUROPEAN PATENT APPLICATION

(11) **EP 2 579 069 A2**
(43) Date of publication of application: **10.04.2013**
(21) Application number: 11790034.0
(22) Date of filing: 03.06.2011
(51) Int. Cl.: G01V 15/00, A61F 13/42, A61F 13/44, H04B 7/00

(54) **DEFECATION/URINATION DETECTION SYSTEM AND METHOD**

(30) Priority: 02.06.2011 KR 20110053433; 04.06.2010 KR 20100052903
(71) Applicant: DTron Co., Ltd., Incheon 406-840 (KR)
(72) Inventor: AHN, Sung Hoon, Bucheon-si Gyeonggi-do 420-021 (KR)
(74) Representative: Peter, Julian
(86) International application number: PCT/KR2011/004065
(87) International publication number: WO 2011/152676

(57) **Abstract**

The present invention relates to detection of defecation/urination of human bodies. In particular, the present invention provides a defecation/urination detection system and method using an RFID passive tag and a reader to measure a change in the sensitivity (or receiving count) of the tag and then inform a user of the incidence in real time mode. The defecation/urination detection system comprises: a first step of registering a defecation/urination detection tag in one-to-one correspondence upon receiving a request for registration of the defecation/urination detection tag, following the initiation; a second step of detecting an energy level of a received signal that is generated from the registered defecation/urination detection tag, thereby detecting sensitivity based on the energy level; and a third step of analyzing the detected sensitivity and then making an alarm sound and a display if there is a change in the sensitivity of the defecation/urination detection tag due to defecation/urination, regarding the change as a criterion for determination of the incidence of defecation/urination.

## Description

### Technical Field

The present invention relates to the detection of defecation/urination and, more particularly, to a defecation/urination detection system and method that, in the detection of the defecation/urination of a human, detect the state of defecation/urination using an RFID method and then notify a user of the results of the detection.

### Background Art

In general, persons who cannot recognize defecation/urination or cannot deal with defecation/urination alone, such as an infant, an elder with dementia, and a critical patient who cannot move about freely, are faced with the situation of dealing with excretions with the help of a guardian or a caregiver. Accordingly, the guardian or caregiver deals with excretions using a method of making an infant, an elder with dementia, or a critical patient, or the like wear a diaper or underwear, frequently checking defecation/urination, and replacing the diaper or underwear.

In such a case, the guardian or caregiver suffers from the inconvenience of frequently checking the infant, the elder with dementia, the critical patient or the like for defecation and urination by the eye in order to determine whether defecation and urination have occurred, and faces the managing difficulty of periodically checking a plurality of patients when managing the plurality of patients in a hospital room. While in order to mitigate these problems, an apparatus equipped with a defecation/urination detection sensor was conceived of and a method of wirelessly monitoring the state of defecation/urination using a sensor (humidity detection sensor) so that a guardian can easily become aware of the state was proposed, it is problematic in that the performance of detection varies depending on the amount of humidity.

A technology proposed to overcome the problem is an RFID tag including a humidity-activated battery. That is, a method in which the tag operated as a passive tag in a normal situation and the tag operated as a self-powered active tag upon defecation/urination and then monitored the state of defecation/urination was proposed.

However, the conventional technology is subjected to the inconvenience of assigning an ID unique number to each tag in order to identify each patient or newly assigning an ID upon replacing each tag in a hospital room in which a plurality of patients is present, and has the disadvantage of undergoing a detection error attributable to battery consumption in the case of a patient who has no defecation/urination for a long time because it includes a low power battery.

Furthermore, the active tag including a battery has the disadvantage of having burden in terms of cost due to an increase in the cost of products when it is frequently used in a disposable fashion.

Furthermore, although a diaper that was equipped with a sensor for detecting defecation/urination and issued an alarm when defecation/urination was detected was developed as another conventional technology, this diaper is configured such that a disposable diaper is provided with an alarm generation device, so that it has the disadvantages of suffering from the inconvenience of attaching the device to the human body, an increase in the cost of a product, and the complexity of manufacturing thereof.

### Disclosure

### Technical Problem

Accordingly, the present invention has been proposed not only to overcome the various problems occurring in the conventional defecation/urination detection method but also to conveniently detect defecation/urination.

An object of the present invention is to provide a defecation/urination detection system and method that detect the state of defecation/urination on a diaper using an RFID method and notify a user (guardian) of the results of the detection.

Another object of the present invention is to provide a defecation/urination detection system and method that detect defecation/urination by measuring a change in tag signal sensitivity (reception count) attributable to the difference between a normal state and the state of defecation/urination using an RFID method and notify a guardian or a caregiver (user) of the results of the detection.

A further object of the present invention is to provide a defecation/urination detection system and method in which a defecation/urination detection tag is fabricated in the form of a passive tag, is fabricated in a disposable form so that it can be easily attached and detached, and is enabled to be attached to a diaper or underwear and then used as desired, thereby achieving convenience in use.

Yet another object of the present invention is to provide a defecation/urination detection system and method that enable a reader and a tag to be registered using an one-to-one automatic registration method for a single corresponding target person in a method of assigning or registering an individual ID in order to identify the relationship between a tag and a target person in a hospital room environment, without manually assigning a target ID upon replacement when there is a plurality of defecation/urination target detection persons, thereby achieving convenience in the registration of a tag.

Still another object of the present invention is to provide a defecation/urination detection system and method that detect the state of the detection of defecation/urination, notify a guardian or a caregiver of the results of the detection using the light-emitting diode (LED) or alarm of the reader or the like in real time, and enable the state of the detection of defecation/urination to a user device via an RFID reader when a guardian or a caregiver is not at the same location, thereby enabling the guardian or caregiver to become aware of the state of the detection of defecation/urination even when he or she is distanced from a defecation/urination detection target person.

### Technical Solution

In order to accomplish the above objects, a first embodiment of the present invention provides "a defecation/urination detection system," including:
an excretion detection tag configured to detect excretions on a diaper; and
an RF reader configured to exchange a wireless signal with the excretion detection tag, to determine whether the diaper has been contaminated depending on whether the wireless signal has been received, and to perform a diaper state notice function if it is determined that the diaper has been contaminated.
In order to accomplish the above objects, a second embodiment of the present invention provides "a defecation/urination detection system," including:
   a defecation/urination detection tag attached to a diaper or underwear, and configured to detect defecation/urination; and
   an RFID reader configured to exchange a wireless signal with the defecation/urination detection tag, to determine whether defecation/urination has occurred according to an algorithm that detects a change in sensitivity attributable to a state of the defecation/urination, and to perform a notice function if it is determined that defecation/urination has occurred.

In order to accomplish the above objects, a first embodiment of the present invention provides "a defecation/urination detection method," including:
a first step of performing initialization and then registering an excretion detection tag if registration of the excretion detection tag is requested;
a second step of maintaining a detection operation of the excretion detection tag if an operating signal is generated by the registered excretion detection tag;
a third step of, if no operating signal is generated by the registered excretion detection tag, determining that a diaper has been contaminated, and performing control so that an alarm is generated, thereby notifying a guardian of the detection of excretions;
a fourth step of releasing the alarm if release of the alarm is requested by the guardian after the alarm has been generated; and
a fifth step of providing notification of a state of the detection of excretions to the guardian's mobile terminal if release of the alarm has not been requested until a preset specific time has elapsed after the generation of the alarm.

In order to accomplish the above objects, a second embodiment of the present invention provides "a defecation/urination detection method," including:
a first step of performing initialization, and registering a defecation/urination detection tag if registration of the defecation/urination detection tag has been requested according to a low-power tag registration procedure;
a second step of detecting an energy level of a signal generated by the registered defecation/urination detection tag and then detecting a sensitivity;
a third step of analyzing the detected sensitivity, and, if there is a change in sensitivity attributable to defecation/urination, determining that defecation/urination has been detected and generating an alarm and a display;
a fourth step of releasing the generated alarm if release of the alarm has been requested within a preset specific time after the generation of the alarm; and
a fifth step of, if the release of the alarm has not been requested within the preset specific time after the generation of the alarm, notifying a remote user of the detection of the defecation/urination by sending an alarm to a user device that was registered in advance.

### Description of Drawings

Fig. 1 is a diagram showing the configuration of a defecation/urination detection system according to a first embodiment of the present invention;
Fig. 2 is a diagram showing the configuration of an embodiment of the RFID reader of Fig. 1;
Fig. 3 is a diagram showing an embodiment of the excretion detection tag of Fig. 1;
Fig. 4 is a diagram showing the configuration of a defecation/urination detection system according to a second embodiment of the present invention;
Fig. 5 is a diagram showing the configuration of an embodiment of the RFID reader of Fig. 4;
Fig. 6 is a sectional view of the defecation/urination detection tag of Fig. 4;
Fig. 7 is a diagram showing the configuration of a defecation/urination detection system according to a third embodiment of the present invention;
Fig. 8 is a flowchart showing a defecation/urination detection method for a diaper according to a first embodiment of the present invention; and
Figs. 9a and 9b are flowcharts showing a defecation/urination detection method according to a second embodiment of the present invention.

### Best Mode

Preferred embodiments of the present invention will be described in detail below with reference to the accompanying drawings. Prior to the description of the present invention, it is noted that detailed descriptions of related well-known functions or configurations will be omitted if they have been deemed to make the gist of the present invention unnecessarily vague.

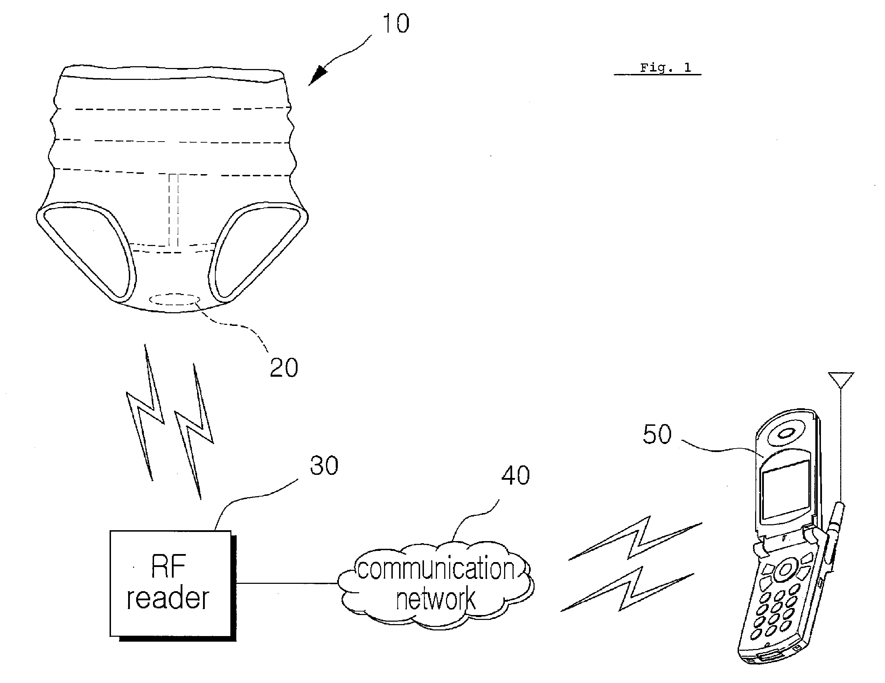

Fig. 1 is a diagram showing the configuration of a "defecation/urination detection system" according to a first embodiment of the present invention, which includes an excretion detection tag 20 attached to a diaper 10, an RF reader 30, a communication network 30, and a mobile terminal 50.

The excretion detection tag 20 functions to detect excretions on the diaper 10. It is preferred that the excretion detection tag 20 be manufactured in a disposable form and be attached to the diaper 10 and then used.

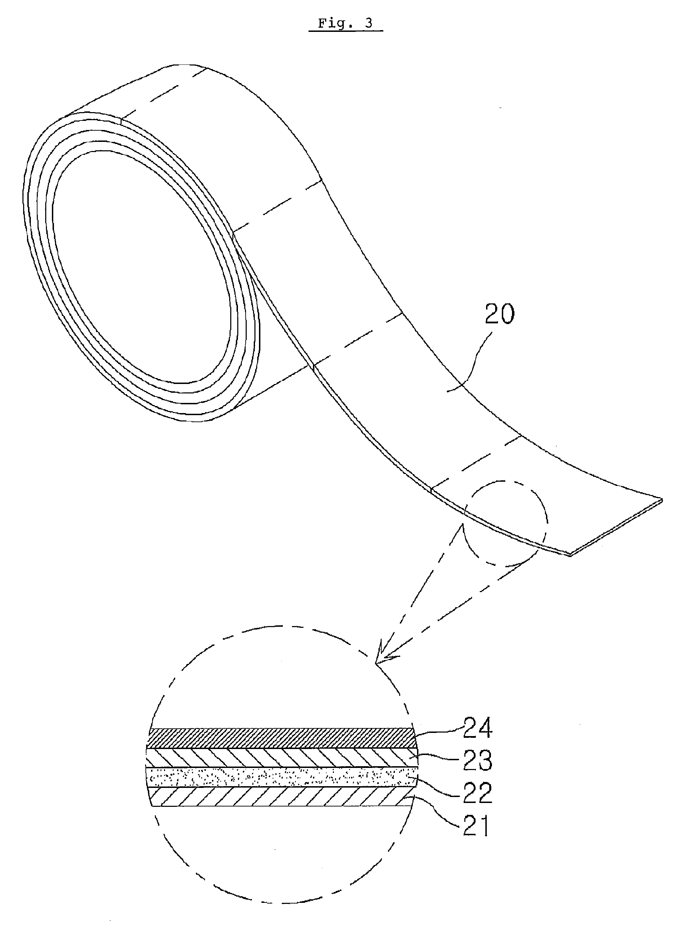

Furthermore, the excretion detection tag 20, as shown in Fig. 3, includes a lower protective film 21; an adhesive part 22 formed on the lower protective film 21; an antenna pattern 23 formed on the adhesive part 22 and configured to detect excretions; and an upper protective film 24 configured to prevent the antenna pattern 23 from being damaged when the antenna pattern 23 is not in use.

The RF reader 30 determines whether excretions have been detected in such a way that the excretion detection tag 20 generates an operating signal when excretions are not detected and enters an operation impossible state and does not generate an operation signal when the excretion detection tag 20 comes into contact with excretions.

The RF reader 30 exchanges wireless signals with the excretion detection tag 20, determines whether the diaper 10 has been contaminated depending on whether the wireless signals have been received, and performs a function of providing notification of the state of the diaper if, as a result of the determination, it is determined that the diaper has been contaminated.

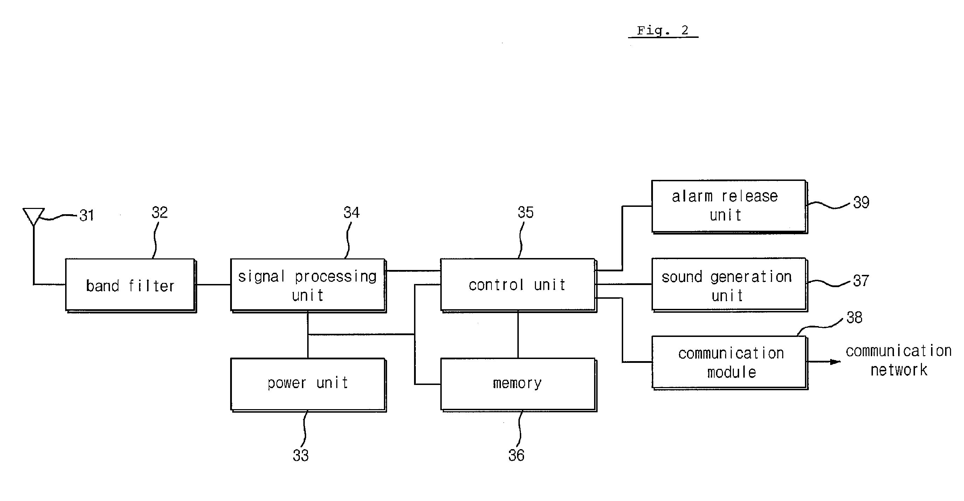

The RF reader 30, as shown in Fig. 2, includes an antenna 31 configured to receive a transmission signal from the excretion detection tag 10, a band filter 32 configured to filter the signal received via the antenna 31 based on a specific band, a signal processing unit 34 configured to process the signal passed through the band filter 34 into digital data, a power unit 33 configured to supply power, and a control unit 35 configured to perform short-distance wireless communication with the excretion detection tag 20, and to, if an operating signal is not generated by the excretion detection tag 20, determine that the diaper has been contaminated, and perform control so that an alarm generation function is performed and an excretion detection message is created and then sent to a mobile terminal carried by a guardian.

Furthermore, the RF reader 30 further includes a sound generation unit 37 configured to generate a diaper contamination alarm under the control of the control unit 35when excretions are detected, memory 36 configured to contain an operation control program for the detection of excretions and to register contact information entered by a guardian in advance, and a communication module 38 configured to send the message to the communication network 40 under the control of the control unit 35.

The communication network 40 is a network that sends a text message created by the RF reader 30 to the mobile terminal 50 carried by a guardian, and refers to the Internet or a mobile communication network.

In the excretions detection apparatus for a diaper according to the present invention, the disposable excretion detection tag 20 intended to be attached to the diaper 10 and then used is separately manufactured in the form of a roll and is cut into pieces and used as desired, as shown in Fig. 3.

If the excretion detection tag 20 is manufactured separately from the diaper 10, there is the advantage of the diaper being able to detect excretions without adding an additional process to an existing diaper manufacturing process in order to detect excretions.

When the excretion detection tag 20 is used, a single excretion detection tag 20 is cut away from a roll along a cutting line, and is registered with the RF reader 30 to be used. Here, only when the excretion detection tag 20 is brought close to the RF reader 30 is RF communication performed between the RF reader 30 and the excretion detection tag 20, and thus it is possible to determine that registration has been performed as soon as communication is performed between the RF reader 30 and the excretion detection tag 20.

Thereafter, the adhesive part 22 is exposed to the outside by removing the lower protective film 21, and the excretion detection tag 20 is attached to a specific location of the diaper 10, such as that shown in Fig. 1, using the adhesive part 22. Here, it is preferable to select as the specific location a location approximate to a location where excretions are discharged from the human body in order to optimally monitor excretions.

The antenna pattern 23 is exposed to the outside by removing the upper protective film 24 with the excretion detection tag 20 attached to the diaper 10, and thus the excretion detection tag 20 is allowed to monitor excretions. Here, the upper protective film 24 is intended to, when the excretion detection tag 20 is not in use, prevent the antenna pattern 23 from being exposed to the outside and being damaged by an external risk (physical force, or contamination caused by a contaminant). It is preferred that the above-described antenna pattern 23 be made of material that is harmless to the human body.

When excretions are not detected in the state in which the antenna pattern 23 is exposed to the outside, an operating signal is generated and thus communication is performed between the excretion detection tag 20 and the RF reader 30. In contrast, when excretions are generated, the excretions come into the antenna pattern 23 and thus the antenna pattern 23 is damaged. Here, it is preferred that the antenna pattern 23 is manufactured such that it will be damaged when it comes into contact with components such as excretions. When excretions are detected, the generation of an operating signal is made impossible by damage to the antenna pattern 23, and accordingly the operating signal of the excretion detection tag 20 is not received by the RF reader 30.

Here, the RF reader 30 receives an operating signal sent by the excretion detection tag 20 via the antenna 31, passes only a set band using the band filter 32, converts the filtered signal into digital data using the signal processing unit 34 via signal processing, and transfers the digital data to the control unit 35, as shown in Fig. 2.

The control unit 35 determines whether the diaper has been contaminated depending on whether digital data is received. As described above, when excretions are not detected, digital data is continuously transferred, and thus the control unit 35 determines that excretions have not been detected. In contrast, if digital data is not received, the control unit 35 determines that excretions have been detected and the diaper has been contaminated.

If it is determined that excretions have been detected and the diaper has been contaminated, the control unit 35 controls the sound generation unit 37 so that a diaper contamination sound (alarm) is generated.

Here, after the sound has been generated, the passage of time is checked using an internal timer. If the release of the alarm is requested using the alarm release unit 39 during the checking of the passage of time, the generation of the alarm is immediately stopped. Since this is the case in which a guardian becomes aware of the alarm and then artificially releases the alarm, the generation of the alarm can be rapidly released.

Meanwhile, if during the checking of the passage of time, the release of the alarm is not requested by a guardian even when a preset time has elapsed, this is determined to be the case in which a guardian is distanced from a wearer who is wearing the diaper, the memory 36 is searched, and then contact information registered by the guardian in advance is extracted.

Thereafter, a text message related to the detection of excretions is created based on information stored in the memory 36, the text message and the contact information are combined together, and the text message is sent to the communication network 40 via the communication module 38.

The text message sent over the communication network 40 is transferred to the mobile terminal 50 carried by a user and displayed on a screen. Accordingly, even a guardian who is away from a wearer who is wearing the diaper can become aware of the situation of the detection of excretions via the mobile terminal 50 carried by him or her. Accordingly, a follow-up action is rapidly taken, and thus the wearer is prevented from experiencing inconvenience attributable to the excretions and from having his or her skin damaged by the excretions.

The above-described present invention has not only the advantage of facilitating the detection of excretions because the disposable excretion detection tag is separately manufactured, attached to a diaper and then used as desired and excretions on a diaper are detected based on the tag-reader concept using an RFID method, but also the advantage of being able to maintain an existing diaper manufacturing process without any changes because the method of attaching the excretion detection tag to a diaper is used. In particular, even when the separately fabricated disposable excretion detection tag is attached to any diaper or then used, excretions can be detected, so that convenience in use is achieved.

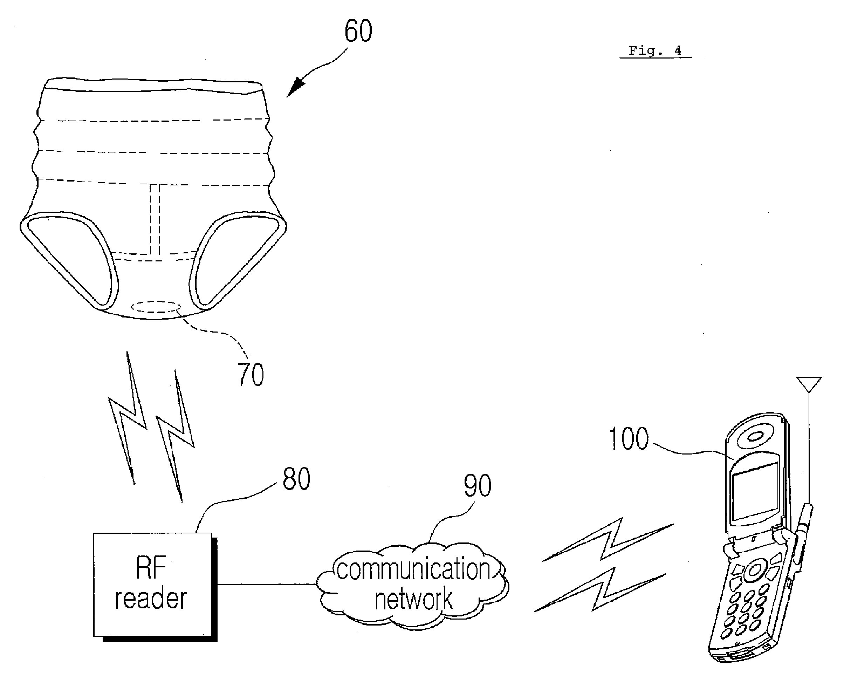

Fig. 4 is a diagram showing the configuration of a defecation/urination detection system according to the second embodiment of the present invention, which includes a wearing target 60 configured such that a defecation/urination detection tag 70 is attached thereto, an RFID reader 80, a communication network 90, and a user device 100.

Here, the wearing target 60 refers to a diaper or underwear that is worn by an infant, an elder with dementia, a critical patient, or the like. The defecation/urination detection tag 70 functions to detect defecation/urination, has a unique ID, is fabricated in a disposable form, and is attached to the wearing target 60 and then used.

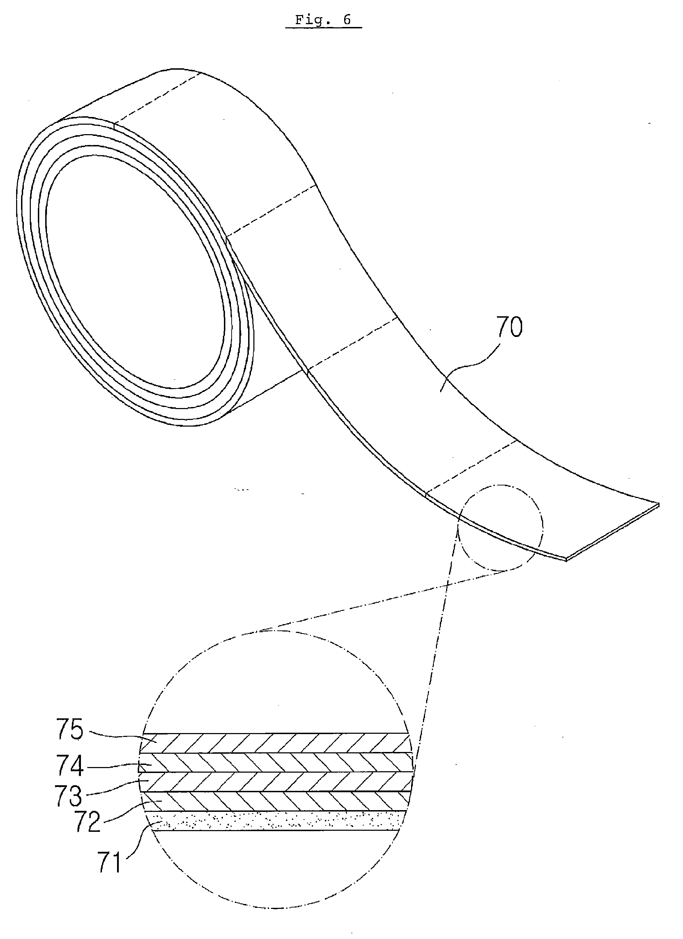

The defecation/urination detection tag 70, as shown in Fig. 6, includes an adhesive part 71 configured to be attached to the wearing target 60, a lower protective film 72, an antenna pattern 73 formed on the lower protective film 72 and configured to detect defecation/urination, a tag chip 74 attached onto the antenna pattern 73 and configured to be responsible for exchanging wireless signals with the RFID reader, and an upper protective member 75 made of soft material, such as non-woven fabric or cotton, in order to prevent the antenna pattern 73 and the tag chip 74 from being damaged and avoid providing eczema or displeasure to the skin when it comes into contact with the skin. Fig. 6 merely shows an embodiment of the defecation/urination detection tag 70. The defecation/urination detection tag 70 may be fabricated in a plat form, other than the above form, depending on a manufacturing method. The defecation/urination detection tag 70 may be implemented in a configuration other than the configuration shown in Fig. 6. Accordingly, the defecation/urination detection tag 70 according to the present invention is not limited to the tag shown in Fig. 6.

The RFID reader 80 exchanges wireless signals with the defecation/urination detection tag 70, determines defecation/urination by detecting a change in sensitivity attributable to the difference between the normal state of the wireless signal and the state of defecation/urination, and performs a notice provision function if it is determined that defecation/urination has occurred.

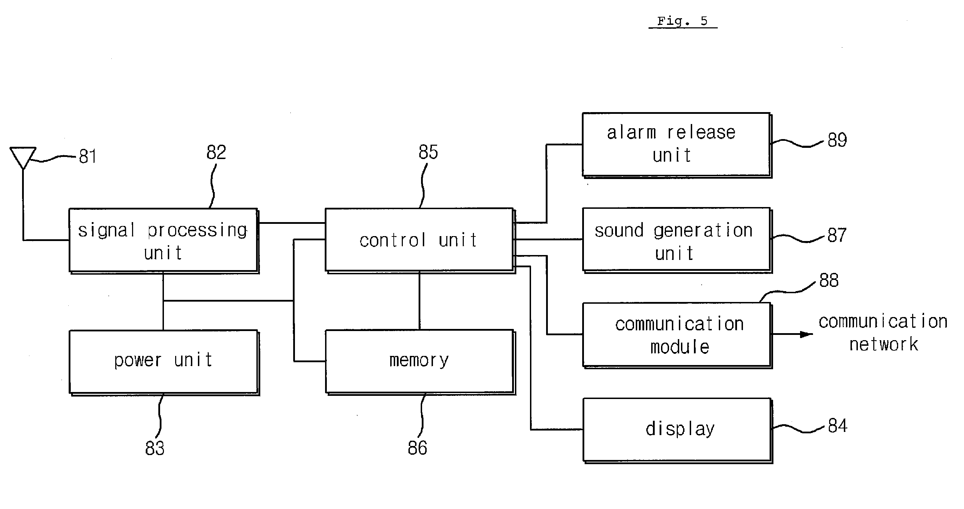

The RFID reader 80, as shown in Fig. 5, includes an antenna 81 configured to receive a transmission signal output from the defecation/urination detection tag 70, a signal processing unit 82 configured to filter the signal received via the antenna 81 based on a specific band, to perform signal processing the filtered signal, and to output results as a defecation/urination sensitivity value, a power unit 83 configured to supply power, a control unit 85 configured to perform short-distance wireless communication with the defecation/urination detection tag 70, to determine whether there is a change in sensitivity by comparing the sensitivity value output from the signal processing unit 84 with a reference sensitivity value stored in memory 86, to determine that defecation/urination has been detected if, as a result of the determination, there is a change in sensitivity, and to control the display of the detection of defecation/urination and the generation of an alarm, and a sound generation unit 87 configured to generate a defecation/urination detection alarm under the control of the control unit 85.

Furthermore, the RFID reader 80 further includes memory 86 configured such that contact information input by a guardian or a caregiver (user) in advance has been registered and a reference sensitivity value used to determine a change in sensitivity has been stored, an alarm release unit 89 configured to release a generated alarm under the control of the control unit 85, and a communication module 88 configured to send the state of the detection of the defecation/urination over the communication network 90 under the control of the control unit 85, thereby sending the state to the remote user device.

Furthermore, the RFID reader 80 further includes a display 84 that visually displays the detection of defecation/urination.

The communication network 90 is a wireless network that is used to send a text message or an alarm signal created by the RFID reader 80 to the user device 100 that is carried by a guardian or a caregiver (user), and refers to the Internet or a mobile communication network.

The user device 100 is preferably implemented using a mobile phone (including a smart phone), a personal computer (PC), or a portable alarm device. In Fig. 4, only a mobile phone is illustrated for convenience of illustration.

In the second embodiment of the defecation/urination detection system according to the present invention, which is configured as described above, as shown in Fig. 4, the disposable defecation/urination detection tag 70 that is attached to the wearing target 60 and then used is separately manufactured, and is cut into pieces and then used as desired by a user. Here, while the defecation/urination detection tag 70 has been described as being manufactured in the form of a roll as a preferred embodiment of the present invention, the present invention is not limited thereto, but may be manufactured and used in the form of a plat sheet. Furthermore, in order to facilitate the use of a user, the defecation/urination detection tag 70 may be cut into separate pieces in advance and then provided to a user.

If the defecation/urination detection tag 70 is manufactured separately from the wearing target 60, there are the advantage of eliminating a need to add the process of attaching a tag to a wearing target to the existing process of manufacturing the wearing target in order for the wearing target to detect defecation/urination, and the advantage of simply attaching the defecation/urination detection tag 70 to an existing diaper or exiting underwear and then using them.

Here, each defecation/urination detection tag 70 is assigned a unique ID. A method of using the system will now be described in greater detail. A single defecation/urination detection tag 70 that is provided in a form that is disposable and can be conveniently used is registered in the RFID reader 80 by bringing the defecation/urination detection tag 70 into contact with the RFID reader 80. In the registration, the defecation/urination detection tag 70 is assigned a unique ID, and thus a user may register the defecation/urination detection tag 70 with the RFID reader 80 in a one-to-one correspondence. Here, when the tag is registered, it is preferable to register the tag at low power over a short distance. Accordingly, the reader performs transmission and reception only with the registered tag regardless of one or more adjacent other tags. In particular, since RF communication is performed between the RFID reader 80 and the defecation/urination detection tag 70, it may be determined that registration has been achieved as soon as RF communication is performed. Here, a reference sensitivity value that is used between the registered defecation/urination detection tag and the RFID reader 80 has been stored in the RFID reader 80 based on a set algorithm.

Thereafter, the adhesive part 71 is exposed to the outside by removing the bottom release paper, and the adhesive part 71 is attached to the wearing target 60 at a predetermined location (a location where defecation/urination can be detected). Furthermore, in some cases, the method of attaching the tag to a diaper may vary. Here, it is preferred that the predetermined location be close to the location where excretions are expelled from the human body in order to maximally monitor defecation/urination.

After the defecation/urination detection tag 70 has been attached to the wearing target 60, communication is performed between the defecation/urination detection tag 70 and the RFID reader 80, and the signal processing unit 82 of the RFID reader 80 receives a wireless transmission signal generated by the defecation/urination detection tag 70, performs signal processing on the received signal, and then sends the processed signal to the control unit 85 in the form of a wireless signal sensitivity value.

Here, when defecation or urination occurs and excretions come into contact with the antenna pattern 73, the sensitivity of a transmission signal sent via the antenna pattern 73 is significantly reduced. This results from the characteristic of the antenna. The sensitivity of a transmission signal may vary depending on the quantity of excretions. Once excretions have come into contact with the antenna pattern 73, the sensitivity of a transmission signal is reduced.

Accordingly, the signal processing unit 82 of the RFID reader 80 processes a received signal, detects the energy level of the received signal, and transfers the detected energy level to the control unit 85 in the form of a received signal sensitivity value.

The control unit 85 compares the transferred received signal sensitivity value with the reference sensitivity value previously stored in the memory 86, and, if the received signal sensitivity value is equal to the previously stored reference sensitivity value, determines that there is no change in signal sensitivity between the defecation/urination detection tag 70 and the RFID reader 80, and therefore determines that defecation/urination has not been detected.

In contrast, if the received signal sensitivity value is smaller than the previously stored a reference sensitivity value, the control unit 85 determines that defecation or urination has been detected, and controls the sound generation unit 87 so that a defecation or urination detection alarm is generated. Furthermore, the control unit 85 activates the display 84 so that a visual indication of the detection of the defecation or urination is given. Here, it is preferred that the display 84 displays the detection of the defecation or urination using one or more light-emitting diodes (LEDs).

After the defecation/urination detection alarm has been generated, the control unit 85 checks the passage of time using an internal timer. When the release of the alarm is requested via the alarm release unit 89 during the checking of the passage of time, the control unit 85 immediately controls the sound generation unit 87 and the display 84 so that the generation of the alarm and the display is stopped.

If a new defecation/urination detection tag is registered during the generation of the alarm, the generation of the alarm and the display is also stopped. In this case, initialization is performed, and then the operation is switched again to the defecation/urination detection operation.

Meanwhile, if a guardian or a caregiver (user) does not request the release of the alarm even when a preset specific time has elapsed during the checking of the passage of time, it is determined that the guardian or caregiver is distanced from a wearer who wears the defecation/urination detection tag, and the memory 86 is searched for the contact information that was registered by the guardian or caregiver in advance.

Thereafter, a text message indicative of the detection of the excretions is generated based on the information stored in the memory 86, the generated text message is combined with the contact information, and the text message is sent via the communication module 88 over the communication network 0. This case corresponds to the case where the user device 100 is a mobile phone (smart phone). If the user device 100 is an alarm generator, only an alarm generation signal is simply generated and sent.

The text message, notice signal or the like sent over the communication network 90 is transferred to the user device 100 carried by the guardian or caregiver, and thus the guardian or caregiver who is distanced from the wearer who wears the defecation/urination detection tag becomes aware of the detection of defecation/urination using the user device 100 that is carried by him or her. Here, a method of providing notification that a text message has arrived using an alarm or vibrations together with the text message may be used. Thereafter, a follow-up action is rapidly taken, and thus the wearer can be prevented from experiencing inconvenience attributable to the defecation or urination and from having his or her skin damaged by the defecation or urination.

It is apparent that when the guardian or caregiver carries an alarm generator as the user device 100, an alarm is generated, and thus he or she becomes aware of the situation of the detection of the defecation/urination. Thereafter, a follow-up action is rapidly taken, and thus the wearer can be prevented from experiencing inconvenience attributable to the defecation or urination and from having his or her skin damaged by the defecation or urination.

Although the above-described present invention has been described only in conjunction with the case where there are a single defecation/urination detection tag and a single RFID reader, the present invention may be configured such that a plurality of RFID readers and a single (portable) alarm generator are connected and used via RF, Bluetooth or ZigBee communication, and thus a guardian or a caregiver who carries an alarm generator may manage a plurality of target persons (persons wearing defecation/urination detection tags) using a plurality of RFID readers:

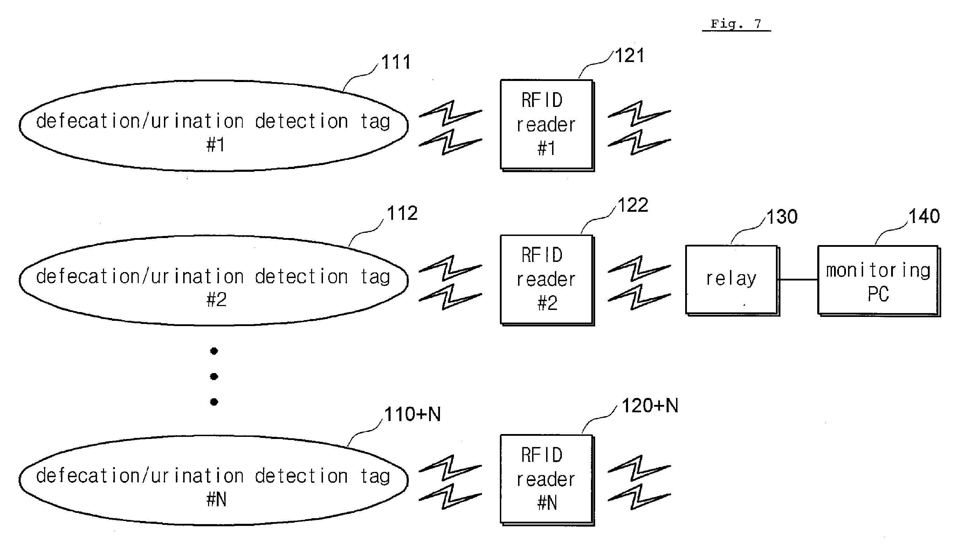

Fig. 7 is a diagram showing the configuration of a defecation/urination detection system according to a third embodiment of the present invention. As shown in this drawing, the defecation/urination detection system may be implemented to include a relay 130 configured to wirelessly exchange defecation/urination detection signals with a plurality of adjacent RFID readers; and a monitoring PC configured to monitor the situations of the detection of defecation/urination detected by the plurality of RFID readers 121 to 120+N in conjunction with the relay 130 in an integrated manner. In this case, the plurality of RFID readers 121 to 120+N that communicates with the plurality of defecation/urination detection tags 111 to 110+N in a one-to-one correspondence is connected to each other via the relay 130, and the relay 130 is connected to the monitoring PC 140, so that a plurality of defecation/urination monitoring tag wearers can be easily managed using the monitoring PC. In this case, the relay and the monitoring PC may be connected and used via a communication method such as ZeeBee, USB, or Bluetooth communication.

The present invention is advantageous in that defecation/urination can be conveniently detected because a disposable defecation/urination detection tag is manufactured and attached to a diaper or underwear and defecation/urination is monitored based on the tag-reader concept using an RFID method, and is advantageous in that an existing wearing target manufacturing process can be used without any change because the method of attaching a defecation/urination detection tag to a diaper or underwear is used. In particular, since defecation/urination can be detected by attaching the separately manufactured disposable tag to the wearing target and using them, significant convenience in use is achieved.

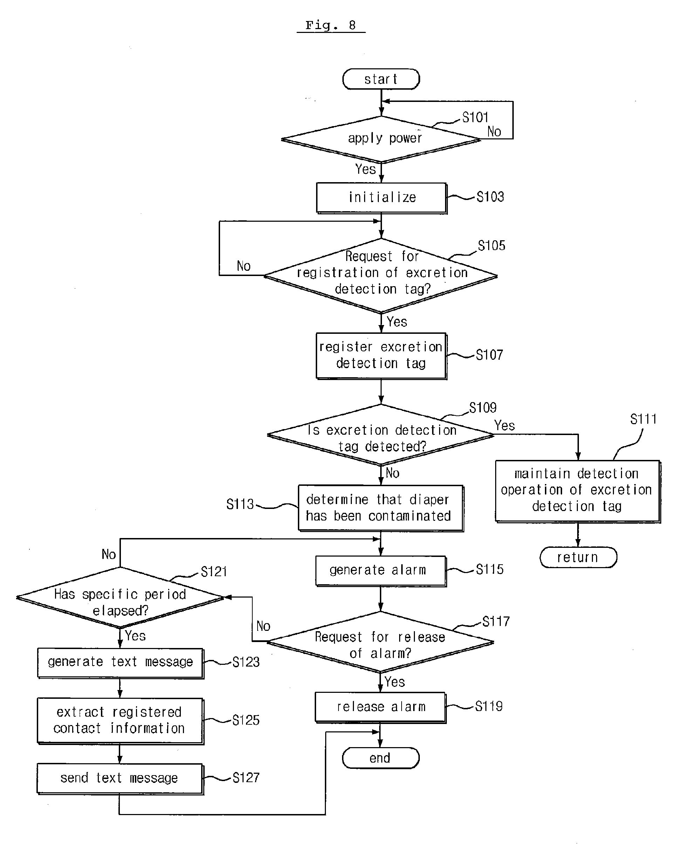

Fig. 8 is a flowchart showing "a defecation/urination detection method" according to a first embodiment of the present invention, in which S refers to step.

As shown in this drawing, the method includes first step S101 to S107 of performing initialization and then registering the excretion detection tag if the registration of the excretion detection tag is requested; second step S109 to S111 of maintaining the detection operation of the excretion detection tag if an operating signal is generated by the registered excretion detection tag; third step S109, and S113 to S115 of, if no operating signal is generated by the registered excretion detection tag, determining that the diaper has been contaminated, and performing control so that an alarm is generated, thereby notifying a guardian of the detection of excretions; fourth step S117 to S119 of releasing the alarm if the release of the alarm is requested by the guardian after the alarm has been generated; and fifth step S121 to S127 of providing notification of the detection of excretions to the guardian's mobile terminal if the release of the alarm has not been requested until a preset specific time has elapsed after the generation of the alarm.

Here, the fifth step includes step S121 of determining whether the preset specific time has elapsed after the generation of the alarm; step S123 of, if, as a result of the determination, the preset specific time has elapsed, creating a text message indicative of the detection of the excretions; step S125 of extracting contact information registered by the guardian in advance; and step S127 of combining the extracted contact information with the text message and sending the text message to the guardian's mobile terminal over the communication network.

When power is applied at step S101, the defecation/urination detection process for a diaper according to the present invention proceeds to step S103, and performs initialization.

If the registration of the excretion detection tag is requested at step S105 after the initialization has been performed, the process proceeds to step S107, and registers the excretion detection tag.

After the excretion detection tag has been registered, it is determined whether an operating signal is generated by the registered excretion detection tag, and, if, as a result of the determination, it is determined that operation signals have been continuously generated by the excretion detection tag, it is determined that excretions have not been detected, and the detection operation of an excretion detection tag is maintained at step S109 to S111.

In contrast, if excretions are generated and the excretion detection tag does not generate an operating signal, it is determined that the diaper has been contaminated at step S113, and an excretion detection alarm is generated at step S115.

After the alarm has been generated, the passage of time is checked, and the generated alarm is immediately released if the release of the alarm is requested by the guardian during the checking of the passage of time at steps S117 to S119.

Meanwhile, if the release of the alarm has not been requested until the specific time preset by the guardian has elapsed during the checking of the passage of time, it is determined that the guardian is distanced from the diaper wearer, and a text message indicative of the contamination of the diaper is created at steps S121 to S123.

Thereafter, contact information registered by the user in advance is extracted from the memory, the extracted contact information is combined with the created text message, and the created text message guardian is sent to a mobile terminal carried by the guardian over a communication network at steps S125 to S127. As a result, there is the advantage of the guardian being able to become aware of the contamination of the diaper worn by the diaper wearer when being distanced from the wearer, and the wearer is prevented from experiencing inconvenience attributable to the contaminated diaper and from having his or her human body damaged by the contaminated diaper by enabling the guardian to take a rapid action when the contamination of the diaper is detected.

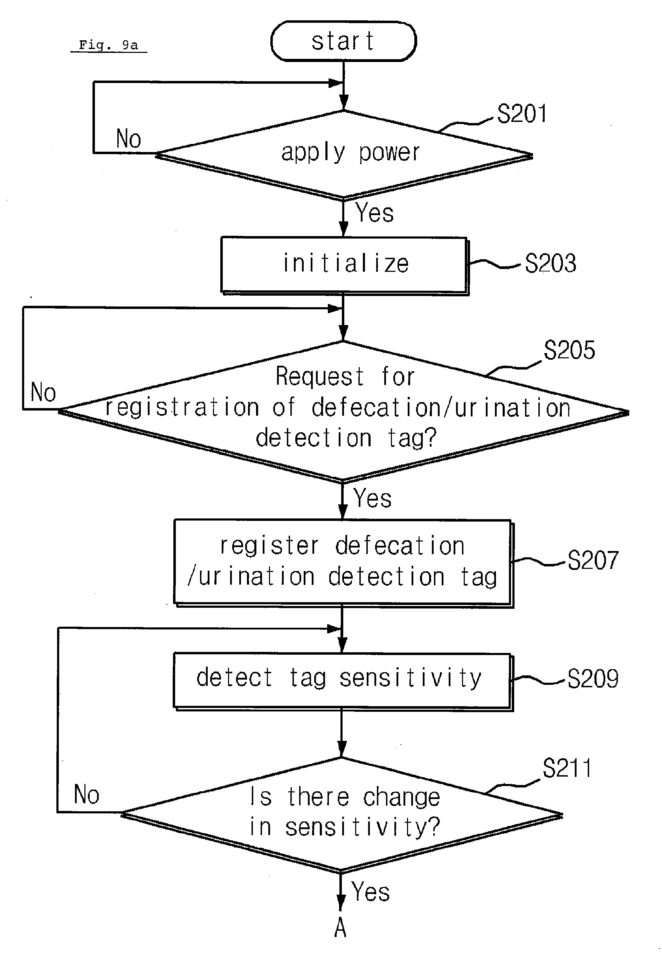

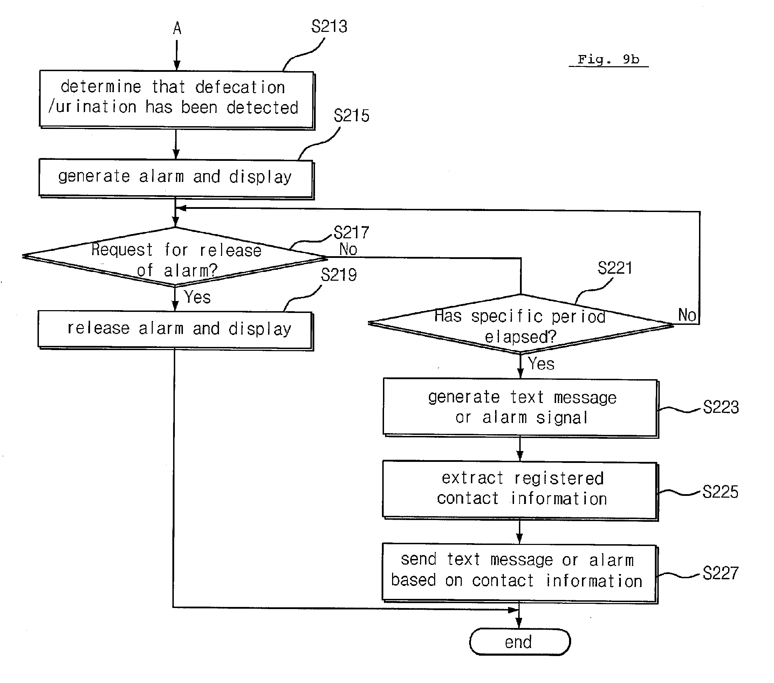

Figs. 9a and 9b are flowcharts showing a defecation/urination detection method according to the present invention, in which S refers to step. This defecation/urination detection method represents a process that is performed in the control unit 85 in a program manner.

As shown in this drawing, the method includes first step S201 to S207 of performing initialization, and registering the defecation/urination detection tag if the registration of the defecation/urination detection tag has been requested; second step S209 of converting a signal received from the registered defecation/urination detection tag into an energy level and then detecting a sensitivity value; third step S211 to S215 of determining whether there is a change in the detected sensitivity, and, if there is a change in the detected sensitivity, determining that defecation/urination has been detected, and providing notification that defecation or urination has been detected by generating an alarm and a display; fourth step S217 to S219 of determining whether the release of the alarm has been requested after the alarm and the display was generated, and releasing the generated alarm and display if the release of the alarm has been requested within the preset specific time; and fifth step S221 to S227 of, if the release of the alarm has not been requested within the preset specific time, generating a text message or an alarm signal, and providing notification of the detection of the defecation/urination by sending the generated text message or alarm signal to the user device.

Here, the fifth step S221 to S227 includes:
step S221 of determining whether the preset specific time has elapsed after the generation of the alarm; step S223 of generating a text message or an alarm signal indicative of the detection of the defecation or urination if, as a result of the determination, the preset specific time has elapsed; step S225 of extracting contact information registered by the guardian or caregiver in advance; and step S227 of combining the extracted contact information with the text message or alarm signal, and sending the text message to the user device carried by the guardian or caregiver over the communication network.

When power is applied at step S201, the defecation/urination detection process for a diaper according to the present invention proceeds to step S203, and performs initialization.

If the registration of the defecation/urination detection tag is requested at step S205 after the initialization has been performed, the process proceeds to step S207, and registers the excretion detection tag.

Here, if the defecation/urination detection tag is manufactured in a disposable form, it has a unique ID.

After registering the tag, the process proceeds to step S209, and receives a transmission signal from the defecation/urination detection tag, performs signal processing on the received signal, detects an energy level, and converts the energy signal into a sensitivity value. Thereafter, it is determined whether there is a change in sensitivity by comparing the resulting sensitivity value with a reference sensitivity value stored in the memory in advance at step S211. Here, if defecation or urination occurs and the antenna pattern is contaminated, the sensitivity of a transmitted signal is significantly reduced. That is, the transmitted signal strength is reduced. Based on this principle, a change in sensitivity can be detected.

If, as a result of the determination of the change in sensitivity, the detected received signal sensitivity value is equal to the reference sensitivity value, it is determined that defecation/urination has not been detected because there is no change in sensitivity, and the defecation/urination detection operation is continuously performed. If the detected sensitivity value is smaller than the reference sensitivity value and there is a change in sensitivity, it is determined that defecation or urination has been detected at step S213.

If it is determined that defecation or urination has been detected, the process proceeds to step S215, and generates an alarm and a display so that the adjacent guardian or caregiver can become aware of the occurrence of the defecation or urination. The guardian or caregiver who becomes aware of it may release the alarm and rapidly take a follow-up action.

In contrast, if the release of the alarm has not be requested within the preset specific time after the alarm and the display was generated, it is determined that the guardian or caregiver determined that the guardian or caregiver is distanced from the wearer of the defecation/urination detection tag, and a text message or an alarm signal indicative of the detection of defecation/urination is generated at steps S221 to S223. Here, the text message is intended to provide notification of the detection of the defecation/urination in the case where the guardian or caregiver carries a mobile phone (smart phone), while the alarm signal is intended to provide notification of the detection of the defecation/urination in the case where the guardian or caregiver carries an alarm generator.

Thereafter, the process proceeds to step S225, and extracts contact information registered by the guardian or caregiver in advance from the memory. At step S227, the extracted contact information is combined with the generated text message or alarm signal, and the message is sent to the user device carried by the guardian or caregiver via the communication network.

As a result, there is the advantage of the guardian or caregiver being able to become aware of the contamination of the wearing target worn by the wearer (defecation/urination) while being distanced from the wearer who wears the defecation/urination detection tag, and the wearer can be prevented from experiencing inconvenience attributable to the contaminated wearing target and from having his or her human body damaged by the contaminated wearing target by enabling the guardian or caregiver to take a rapid action when the contamination of the wearing target is detected. In this case, the guardian or caregiver who is distanced from the wearer may send an alarm release signal using the user device carried by him or her. When the communication module of the RFID reader receives the alarm release signal, the alarm may be automatically released under the control of the control unit. That is, if the alarm is continuously generated when the guardian or caregiver is at a remote location, surrounding persons may feel offensive, and thus the present invention provides the function of releasing the alarm from a remote location in order to eliminate such offensiveness.

The present invention is not limited to the above-described specific preferred embodiments. It is apparent that those having ordinary knowledge in the technical field to which the present invention pertains may modify and practice the present invention in various ways without departing from the gist of the present invention set forth in claims. The modifications fall within the scope of the present invention that is described in the claims.

### Industrial Applicability

The present invention has the advantage of conveniently detecting the state of defecation/urination using an RFID method and notifying a guardian or a caregiver (user) of the results of the detection.

Furthermore, the present invention has the advantage of fabricating the defecation/urination detection tag in a disposable form and enabling the defecation/urination detection tag to be attached to and detached from a diaper or underwear and then used, thereby achieving convenience in use.

Furthermore, the present invention has the advantage of, in a method of notifying a guardian or a caregiver (user) of the detection of defecation/urination, sending a text message or a notice signal indicative of the state of defecation/urination to a user device used by the guardian or the like when the release of an alarm has not been requested within a predetermined time, thereby enabling the guardian or caregiver (user) to become aware of the state of defecation/urination when the guardian or caregiver (user) is distanced from a defecation/urination detection tag wearer such as an infant, an elder with dementia, a critical patient, or the like.

Furthermore, the present invention has the advantage of enabling a small number of persons to efficiently manage a plurality of management target persons in a group management environment, such as a hospital room in which an infant, an elder with dementia, a critical patient and/or the like are present together, thereby achieving a reduction in management expenses.

## Claims

1. A defecation/urination detection system, comprising:
an excretion detection tag configured to detect excretions on a diaper; and
an RF reader configured to exchange a wireless signal with the excretion detection tag, to determine whether the diaper has been contaminated depending on whether the wireless signal has been received, and to perform a diaper state notice function if it is determined that the diaper has been contaminated.

2. The defecation/urination detection system of claim 1, wherein the excretion detection tag comprises:
a lower protective film;
an adhesive part formed on the lower protective film;
an antenna pattern formed on the adhesive part, and configured to detect excretions; and
an upper protective film configured to prevent the antenna pattern from being damaged when the antenna pattern is not in use.

3. The defecation/urination detection system of claim 1, wherein the excretion detection tag, when coming into contact with excretions, enters an operation impossible state and does not generate an operation signal.

4. The defecation/urination detection system of claim 1, wherein the RF reader comprises:
a control unit configured to perform short-distance wireless communication with the excretion detection tag, and to, if an operating signal is not generated by the excretion detection tag, determine that the diaper has been contaminated and perform control so that an alarm generation function is performed and an excretion detection message is created and then sent to a mobile terminal carried by a guardian;
a sound generation unit configured to generate a diaper contamination alarm under a control of the control unit if excretions are detected; and
a communication module configured to send the message to a communication network under a control of the control unit.

5. A defecation/urination detection system, comprising:
a defecation/urination detection tag attached to a diaper or underwear, and configured to detect defecation/urination; and
an RFID reader configured to exchange a wireless signal with the defecation/urination detection tag, to determine whether defecation/urination has occurred according to an algorithm that detects a change in sensitivity attributable to a state of the defecation/urination, and to perform a notice function if it is determined that defecation/urination has occurred.

6. The defecation/urination detection system of claim 5, wherein the defecation/urination detection tag has a unique ID, is fabricated in a disposable form, is attached to the diaper or underwear and then used, and exchanges the signal with the RFID reader in a one-to-one correspondence.

7. The defecation/urination detection system of claim 5, wherein the defecation/urination detection tag comprises:
a lower protective film;
an antenna pattern formed on the lower protective film, and configured to detect defecation/urination;
a tag chip attached onto the antenna pattern, and configured to be responsible for exchanging a wireless signal with the RFID reader.

8. The defecation/urination detection system of claim 7, wherein the antenna pattern has sensitivity of transmission and reception that varies if defecation/urination is detected.

9. The defecation/urination detection system of claim 5, further comprising:
a relay configured to wirelessly exchange defecation/urination detection signals with the RF reader and a plurality of adjacent RFID readers; and
a monitoring personal computer (PC) configured to monitor a state of detection of defecation/urination detected by the plurality of RFID readers in conjunction with the relay in an integrated manner

10. The defecation/urination detection system of claim 5, wherein the RF reader comprises:
a control unit configured to perform short-distance wireless communication with the defecation/urination detection tag, to determine that defecation/urination has been detected if there is a change in sensitivity from the defecation/urination detection tag, and to control a display of the detection of defecation/urination and generation of an alarm;
a sound generation unit configured to generate a defecation/urination detection alarm under a control of the control unit;
a communication module configured to send the state of the detection of the defecation/urination over the communication network under a control of the control unit, thereby sending the state to a remote user device; and
a display configured to visually display the state of the detection of defecation/urination under a control of the control unit.

11. A defecation/urination detection method, comprising:
a first step of performing initialization and then registering an excretion detection tag if registration of the excretion detection tag is requested;
a second step of maintaining a detection operation of the excretion detection tag if an operating signal is generated by the registered excretion detection tag;
a third step of, if no operating signal is generated by the registered excretion detection tag, determining that a diaper has been contaminated, and performing control so that an alarm is generated, thereby notifying a guardian of the detection of excretions;
a fourth step of releasing the alarm if release of the alarm is requested by the guardian after the alarm has been generated; and
a fifth step of providing notification of a state of the detection of excretions to the guardian's mobile terminal if release of the alarm has not been requested until a preset specific time has elapsed after the generation of the alarm.

12. A defecation/urination detection method, comprising:
a first step of performing initialization, and registering a defecation/urination detection tag if registration of the defecation/urination detection tag has been requested according to a low-power tag registration procedure;
a second step of detecting an energy level of a signal generated by the registered defecation/urination detection tag and then detecting a sensitivity; and
a third step of analyzing the detected sensitivity, and, if there is a change in sensitivity attributable to defecation/urination, determining that defecation/urination has been detected and generating an alarm and a display.

13. The defecation/urination detection method of claim 12, further comprising:
a fourth step of releasing the generated alarm if release of the alarm has been requested within a preset specific time after the generation of the alarm.

14. The defecation/urination detection method of claim 13, further comprising:
a fifth step of, if the release of the alarm has not been requested within the preset specific time after the generation of the alarm, notifying a remote user of the detection of the defecation/urination by sending an alarm to a user device that was registered in advance.

15. The defecation/urination detection method of claim 14, wherein the fifth step comprises steps of:
determining whether the preset specific time has elapsed after the generation of the alarm;
generating a text message or an alarm signal indicative of the detection of the defecation or urination if, as a result of the determination, the preset specific time has elapsed;
extracting contact information registered by a guardian in advance; and
combining the extracted contact information with the text message or alarm signal, and sending the text message or the alarm signal to the user device carried by the guardian over a communication network.
